(19) Europäisches Patentamt
European Patent Office
Office européen des brevets

(11) Veröffentlichungsnummer: **0 079 856**
**A1**

# (12) EUROPÄISCHE PATENTANMELDUNG

(21) Anmeldenummer: **82810477.8**

(22) Anmeldetag: **08.11.82**

(51) Int. Cl.³: **C 07 D 249/08**
C 07 D 405/00, A 01 N 43/64
//C07C43/174

(30) Priorität: **12.11.81 CH 7273/81**

(43) Veröffentlichungstag der Anmeldung:
**25.05.83 Patentblatt 83/21**

(84) *Benannte Vertragsstaaten:*
**AT BE CH DE FR GB IT LI LU NL SE**

(71) Anmelder: **CIBA-GEIGY AG**
**Patentabteilung Postfach**
**CH-4002 Basel(CH)**

(72) Erfinder: **Sturm, Elmar, Dr.**
**Klusstrasse 66**
**CH-4147 Aesch(CH)**

## (54) Mikrobizide Triazolyl-vinyläther.

(57) Es werden neue 1-Phenyl-2-triazolyl-vinylether de allgemeinen Formel I beschrieben,

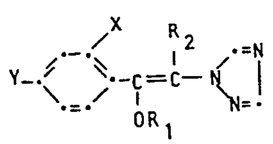

(I),

worin

X für Wasserstoff oder Halogen steht;

Y Wasserstoff, Halogen oder ein gegebenenfalls durch ein oder zwei Halogenatome substituiertes Phenyl bedeutet;

$R_1$ für $C_1$-$C_8$-Alkyl, $C_3$-$C_5$-Alkenyl, $C_3$-Alkinyl, einen Niederalkoxyalkylrest mit maximal 8 Kohlenstoffatomen oder für eine der Gruppen

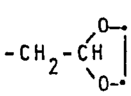 oder 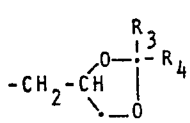 steht,

wobei $R_3$ und $R_4$ unabhängig voneinander Wasserstoff oder Methyl bedeuten; und $R_2$ für Wasserstoff, $C_1$-$C_4$-Alkyl, $C_3$-Alkenyl, $C_3$-Alkinyl oder ein gegebenenfalls ein- oder zweifach durch Halogen, $C_1$-$C_3$-Alkyl oder $C_1$-$C_3$-Alkoxy substituiertes Phenyl steht; unter Einschluss ihrer Säureadditionssalze, quaternären Azoliumsalze und Metallkomplexe.

Es werden ferner Methoden zur Herstellung dieser Produkte offenbart sowie agrochemische Schädlingsbekämpfungsmittel, die als Wirkstoff eine dieser Verbindungen enthalten. Ferner wird ein Verfahren zur Bekämpfung phytopathogener Mikroorganismen mit Hilfe dieser Substanzen beschrieben.

EP 0 079 856 A1

Croydon Printing Company Ltd

CIBA-GEIGY AG 5-13656/=

Basel (Schweiz)

Mikrobizide Mittel

Die vorliegende Erfindung betrifft neue, substituierte 1-Phenyl-3-triazolyl-vinylether sowie deren Säureadditionssalze, quaternäre Azoliumsalze und Metallkomplexe. Die Erfindung betrifft ferner die Herstellung dieser Substanzen sowie mikrobizide Mittel, die als Wirkstoff mindestens eine dieser Verbindungen enthalten. Die Erfindung betrifft auch die Herstellung der genannten Mittel und die Verwendung der Wirkstoffe oder der Mittel zur Bekämpfung von schädlichen Mikroorganismen.

Es werden hierin Verbindungen der allgemeinen Formel I

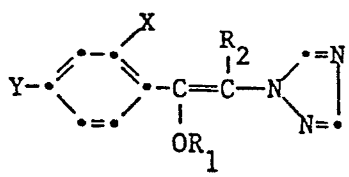 (I),

beschrieben, worin

X für Wasserstoff oder Halogen steht;

Y Wasserstoff, Halogen oder ein gegebenenfalls durch ein oder zwei Halogenatome substituiertes Phenyl bedeutet;

$R_1$ für $C_1$-$C_8$-Alkyl, $C_3$-$C_5$-Alkenyl, $C_3$-Alkinyl, einen Niederalkoxyalkylrest mit maximal 8 Kohlenstoffatomen oder für eine der Gruppen

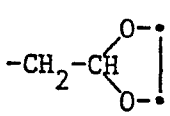 oder 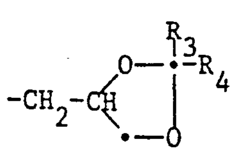 steht,

wobei $R_3$ und $R_4$ unabhängig voneinander Wasserstoff oder Methyl bedeuten; und $R_2$ für Wasserstoff, $C_1$-$C_4$-Alkyl, $C_3$-Alkenyl, $C_3$-Alkinyl oder ein gegebenenfalls ein- oder zweifach durcb Halogen, $C_1$-$C_3$-Alkyl oder $C_1$-$C_3$-Alkoxy substituiertes Phenyl steht; unter Einschluss ihrer Säureadditionssalze, quaternären Azoliumsalze und Metallkomplexe.

Unter dem Begriff Alkyl selbst oder als Bestandteile eines anderen Substituenten sind je nach der Anzahl der angegebenen Kohlenstoffatome beispielsweise eine der folgenden Gruppen zu verstehen: Methyl, Ethyl, Propyl, Butyl, Pentyl, Hexyl, Heptyl, Octyl, usw. sowie ihre Isomeren, wie z.B. Isopropyl, Isobutyl, tert.-Butyl, sek. Butyl, Isopentyl usw. Unter Halogen soll hier und im folgenden Fluor, Chlor, Brom oder Jod, vorzugsweise Fluor, Chlor oder Brom verstanden werden. Alkenyl bedeutet z.B. Propenyl-(1), Allyl, Butenyl-(1), Butenyl-(2), Butenyl-(3), Pentenyl-(2) usw. Alkinyl steht insbesondere für Propargyl. Unter Niederalkoxyalkyl sollen z.B. folgende Substituenten verstanden werden: $-CH_2OCH_3$, $-CH_2-CH_2-OCH_3$, $-CH_2-OC_2H_5$, $-CH_2-OC_3H_7$, $-CH_2-OC_7H_{15}$, $-CH_2-CH_2-O-C_2H_5$, $-CH_2-CH_2-CH_2-OC_3H_7$, $-CH_2-CH_2-CH_2-OC_4H_9$ usw.

Die vorliegende Erfindung betrifft somit die freien organischen Moleküle der Formel I, deren Säureadditionssalze, quaternären Azoliumsalze und Metallkomplexe, wobei die freien Moleküle bevorzugt sind.

Beispiele salzbildender Säuren sind anorganische Säuren: Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, Phosphorige Säure, Salpetersäure und organische Säuren wie Essigsäure, Trilfuoressigsäure, Trichloressigsäure, Propionsäure, Glykolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure oder 2-Acetoxybenzoesäure.

Metallkomplexe der Formel I bestehen aus dem zugrundeliegenden organischen Molekül und einem anorganischen oder organischen Metallsalz, beispielsweise den Halogeniden, Nitraten, Sulfaten, Phosphaten, Acetaten, Trifluoracetaten, Trichloracetaten, Propionaten, Tartraten, Sulfonaten, Salicylaten, Benzoaten usw. der Elemente der dritten und vierten Hauptgruppe wie Aluminium, Zinn oder Blei sowie der ersten bis achten Nebengruppe wie Zirkonium, Chrom, Mangan, Eisen, Kobalt, Nickel, Kupfer, Zink, Silber, Quecksilber usw. Bevorzugt sind die Nebengruppen-Elemente der 4. Periode. Die Metalle können dabei in den verschiedenen ihnen zukommenden Wertigkeiten vorliegen. Die Metallkomplexe der Formel I können ein- oder mehrkernig auftreten, d.h. sie können ein oder mehrere organische Molekülanteile als Liganden enthalten. Komplexe mit den Metallen Kupfer, Zink, Mangan, Zirkonium und Zinn sind bevorzugt.

Die Verbindungen der Formel I sind bei Raumtemperatur stabile Oele, Harze oder überwiegend Feststoffe, die sich durch sehr wertvolle mikrobizide Eigenschaften auszeichnen. Sie lassen sich auf dem Agrarsektor oder verwandten Gebieten präventiv und kurativ zur Bekämpfung von pflanzenschädigenden Mikroorganismen einsetzen. Die erfindungsgemässen Wirkstoffe der Formel I zeichnen sich in weiten Anwendungskonzentrationen durch eine hervorragende mikrobizide Wirkung und problemlose Anwendbarkeit aus.

Aufgrund ihrer ausgeprägten mikrobiziden Wirkung sind diejenigen Wirksubstanzen der Formel I bevorzugt, die folgende Substituententypen oder Kombinationen dieser Substituenten untereinander aufweisen:

Bei X:
- a) H, F, Cl, Br, J
- b) H, F, Cl, Br,
- c) H, Cl
- d) Cl

Bei Y:
a) H, F, Cl, Br, J, $C_6H_5$, $C_6H_4Hal(2)$, $C_6H_4Hal(Y)$, $C_6H_3Hal_2(2,4)$, $C_6H_3Hal_2(3,4)$ mit Hal = F, Cl, Br, J
b) H, F, Cl, $C_6H_5$, $C_6H_4Cl(2)$, $C_6H_4Cl(4)$, $C_6H_3Cl_2(2,4)$, $C_6H_3Cl_2(3,4)$
c) H, F, Cl, $C_6H_5$
d) F, Cl

Bei $R_1$:
a) $C_1$-$C_5$-Alkyl, $CH_2CH{=}CH_2$, $CH_2C{\equiv}CH$, $CH_2OCH_3$, $CH_2OC_2H_5$, $CH_2OC_3H_7$, $CH_2CH_2OCH_3$, $CH_2CH_2OC_2H_5$,
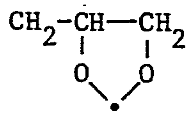 , 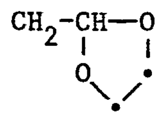 , 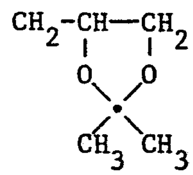
b) $C_1$-$C_4$-Alkyl, $CH_2CH{=}CH_2$, $CH_2C{\equiv}CH$, $CH_2OCH_3$, $CH_2OC_2H_5$ $CH_2CH_2OCH_3$
c) $CH_3$, $C_2H_5$, $C_3H_7(n)$, $C_3H_7(i)$, $C_4H_9(n)$, $C_4H_9(s)$, $C_4H_9(t)$, $CH_2OCH_3$, $CH_2OC_2H_5$, $CH_2CH_2OCH_3$
d) $CH_3$, $C_2H_5$, $C_3H_7(n)$, $C_4H_9(n)$

Bei $R_2$:
a) H, $C_1$-$C_4$-Alkyl, $CH_2CH{=}CH_2$, $CH_2C{\equiv}CH$, $C_6H_3Cl_2(2,4)$, $C_6H_4Cl(4)$
b) H, $C_1$-$C_4$-Alkyl
c) H, $CH_3$, $C_2H_5$, $C_3H_7(n)$, $C_3H_7(i)$, $C_4H_9(n)$, $C_4H_9(s)$
d) H

Es ergeben sich somit z.B. folgende bevorzugten Gruppen a bis d von Verbindungen der Formel I:

a) Verbindungen der Formel I, worin X für Wasserstoff, Fluor, Chlor, Brom oder Jod steht; Y Wasserstoff, Fluor, Chlor, Brom, Jod, $C_6H_5$, $C_6H_4Hal(2)$, $C_6H_4Hal(4)$, $C_6H_3Hal_2(2,4)$ oder $C_6H_3Hal_2(3,4)$ bedeutet, wobei Hal für Fluor, Chlor, Brom oder Jod steht; $R_1$ für $C_1$-$C_5$-Alkyl, $CH_2CH{=}CH_2$, $CH_2C{\equiv}CH$, $CH_2OCH_3$, $CH_2OC_2H_5$, $CH_2OC_3H_7$, $CH_2CH_2OCH_3$,

$CH_2CH_2OC_2H_5$, 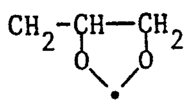, 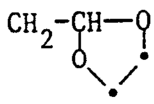 oder 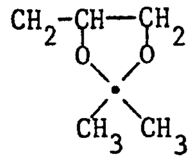

steht und $R_2$ Wasserstoff, $C_1$-$C_4$-Alkyl, $CH_2CH{=}CH_2$, $CH_2C{\equiv}CH$, $C_6H_3Cl_2(2,4)$ oder $C_6H_4Cl(4)$ bedeutet.

b) Verbindungen der Formel I, worin X für Wasserstoff, Fluor, Chlor oder Brom steht, Y Wasserstoff, Fluor, Chlor, $C_6H_5$, $C_6H_4Cl(2)$, $C_6H_4Cl(4)$, $C_6H_3Cl_2(2,4)$ oder $C_6H_3Cl_2(3,4)$ bedeutet; $R_1$ für $C_1$-$C_4$-Alkyl, $CH_2CH{=}CH_2$, $CH_2C{\equiv}CH$, $CH_2OCH_3$, $CH_2OC_2H_5$ oder $CH_2CH_2OCH_3$ steht und $R_2$ Wasserstoff oder $C_1$-$C_4$-Alkyl bedeutet.

c) Verbindungen der Formel I, worin X für Wasserstoff oder Chlor; Y für Wasserstoff, Fluor, Chlor oder $C_6H_5$ stehen; $R_1$ Methyl, Ethyl, n-Propyl, iso-Propyl, n-Butyl, sek.-Butyl, tert.-Butyl, $CH_2OCH_3$, $CH_2OC_2H_5$ oder $CH_2CH_2OCH_3$ bedeutet und $R_2$ für Wasserstoff, Methyl, Ethyl, n-Propyl, iso-Propyl, n-Butyl oder sek.-Butyl steht.

d) Verbindungen der Formel I, worin X für Chlor steht; Y Fluor oder Chlor bedeutet; $R_1$ für Methyl, Ethyl, n-Propyl oder n-Butyl steht und $R_2$ Wasserstoff bedeutet.

Folgende Einzelverbindungen sind besonders bevorzugt:
1-(2,4-Dichlorphenyl)-1-n-propyloxy-2-(1H-1,2,4-triazol-1-yl)-ethen,
1-(2,4-Dichlorphenyl)-1-n-butyloxy-2-(1H-1,2,4-triazol-1-yl)-ethen,
1-(2,4-Dichlorphenyl)-1-(1'-methylbutoxy)-2-(1H-1,2,4-triazol-1-yl)-ethen,
1-(2,4-Dichlorphenyl)-1-n-butyloxy-2-(1H-1,2,4-triazol-1-yl)-buten,
1-(2,4-Dichlorphenyl)-1-n-butoxy-2-(1H-1,2,4-triazol-1-yl)-hexen.

Die Verbindungen der Formel I können nach folgendem Verfahren hergestellt werden, indem man ein α-1H-1,2,4-Triazolyl-phenon der Formel II,

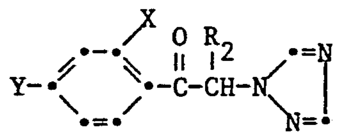

(II),

worin X, Y und $R_2$ die unter Formel I angegebenen Bedeutungen haben, mit einer Verbindung der Formel III,

$R_1$-Z (III),

worin $R_1$ wie unter Formel I definiert ist und Z für eine der folgenden Abgangsgruppierungen -Chlor, -Brom, $R_1SO_4^{\ominus}$ oder $(R_1O)_2(O)P{=}O^{\ominus}$ steht, worin $R_1$ ebenfalls wie unter Formel I definiert ist, in Anwesenheit einer Base und vorteilhafterweise in Anwesenheit eines polaren, aprotischen Lösungsmittels oder eines wässrig-organischen Zweiphasenmediums in Gegenwart eines üblichen Phasentransferkatalysators reagieren lässt und, sofern gewünscht, die freien Moleküle der Formel I nach üblichen Methoden in ihre Säureadditionssalze, quaternären Azoliumsalze oder Metallkomplexe überführt.

Geeignete polare, aprotische Lösungsmittel sind z.B. Dimethylsulfoxid, Dimethylformamid, Hexamethylphosphorsäuretriamid, Dimethylacetamid, N-Methylpyrrolidin, 2-Pyrrolidinon u.a. stark polare, aprotische Solventien. Auch Gemische dieser Solventien mit anderen reaktionsinerten üblichen Lösungsmitteln wie z.B. Benzol, Toluol, Xylole usw. können verwendet werden. In manchen Fällen kann die Verbindung der Formel III selbst als Lösungsmittel eingesetzt werden.

Für die organische, mit Wasser nicht mischbare Phase kommen z.B. folgende Lösungsmittel in Frage: Aliphatische und aromatische Kohlenwasserstoffe, wie Pentan, Hexan, Cyclohexan, Petrolether, Ligroin,

Benzol, Toluol, Xylole usw., halogenierte Kohlenwasserstoffe, wie Dichlormethan, Chloroform, Tetrachlorkohlenstoff, Ethylendichlorid, 1,2-Dichlorethan, Tetrachlorethylen usw. oder aliphatische Ether, wie Diethylether, Diisopropylether, t-Butylmethylether usw..

Zur Beschleunigung der Reaktionsgeschwindigkeit ist bei dieser Durchführungsart die Gegenwart eines Phasentransfer-Katalysators erforderlich. Beispiele derartiger Katalysatoren sind: Tetraalkylammoniumhalogenide, -hydrogensulfate oder -hydroxide wie Tetrabutylammoniumchlorid, -bromid, -jodid; Triethylbenzylammoniumchlorid, -bromid; Tetrapropylammoniumchlorid, -bromid, -jodid; usw.. Als Phasentransfer-Katalysatoren kommen auch Phosphonium-Salze in Betracht.

Die Reaktion wird in Gegenwart geeigneter Basen durchgeführt. Beispiele solcher Basen sind anorganische Basen wie die Oxide, Hydroxide, Hydride, Carbonate und Hydrogencarbonate von Alkali und Erdalkalimetallen sowie organische Basen wie z.B. tert. Amine wie Triethylamin, Triethylendiamin, Piperidin, Pyridin, 4-Dimethylaminopyridin, 4-Pyrrolidylpyridin usw.

Die Reaktionstemperaturen liegen bei der beschriebenen Umsetzung im allgemeinen zwischen 0° und +120°C, vorzugsweise +20° und +80°C.

Man setzt dabei vorzugsweise 1 Mol des α-1H-1,2,4-Triazolylphenons der Formel III mit 1 bis 2 Mol der Verbindung der Formel III ein.

Neben den gewünschten Vinylethern der Formel I können auch Substanzen entstehen, bei denen der Substituent $R_1$ nicht an den Keton-Sauerstoff wandert, sondern ein Wasserstoffatom an der benachbarten Methylengruppe substituiert. Diese Nebenprodukte lassen sich auf einfache Weise, z.B. säulenchromatographisch von den Verbindungen der Formel I abtrennen..

Neben den genannten allgemeinen Herstellungsverfahren kann bei Verbindungen der Formel I, bei denen der Substituent $R_2$ für Wasserstoff steht, und die hier und im folgenden als Untergruppe I' bezeichnet werden, eine andere, spezielle Herstellungsvariante Vorteile bieten. So können die Verbindungen der Formel I' auch dadurch hergestellt werden, dass man ein offenes Acetal der Formel IV

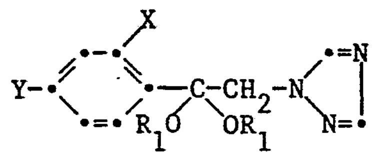

(IV),

worin X, Y und $R_1$ die unter Formel I angegebenen Bedeutungen haben, einer Thermolyse unterwirft und, sofern gewünscht, die freien Moleküle der Formel I nach üblichen Methoden in ihre Säureadditionssalze, quaternären Azoliumsalze oder Metallkomplexe überführt. Diese Thermolyse wird bevorzugt in hochsiedenden, reaktionsinerten Lösungsmitteln bei Temperaturen zwischen +100° und +220°C durchgeführt. Geeignete Lösungsmittel sind z.B. Siliconöl, Mineralöl, "Dowtherm" (= eutektische Mischung von Biphenyl und Dibenzofuran), Diphenylether usw.

Die α-1H-1,2,4-Triazolylphenone der Formel II können z.B. durch Halogenierung der zugrundeliegenden Ausgangsketone V,

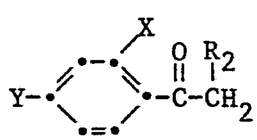

(V),

zu Haloketonen der Formel VI,

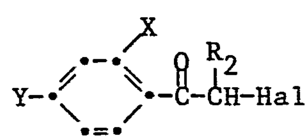

(VI),

und Weiterreaktion von VI mit einem Triazol der Formel VII

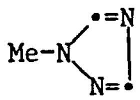 (VII),

hergestellt werden, dabei haben die Substituenten X, Y und $R_2$ die unter Formel I angegebenen Bedeutungen, Hal steht für Halogen, bevorzugt Chlor oder Brom und Me repräsentiert Wasserstoff oder bevorzugt ein Metallatom, insbesondere ein Alkalimetallatom, vorzugsweise Natrium oder Kalium. Die Ausgangsverbindungen V und VII sind allgemein bekannt oder können nach an sich bekannten Methoden hergestellt werden.

Die Reaktion VI mit VII wird bevorzugt in einem relativ polaren, jedoch reaktionsinerten organischen Lösungsmittel durchgeführt, z.B. N,N-Dimethylformamid, N,N-Dimethylacetamid, Dimethylsulfoxid, Acetonitril, Benzonitril und anderen. Derartige Lösungsmittel können in Kombination mit anderen reaktionsinerten Lösungsmitteln, wie aliphatischen oder aromatischen Kohlenwasserstoffen, z.B. Benzol, Toluol, Xylol, Hexan, Petrolether, Chlorbenzol, Nitrobenzol u.a. verwendet werden.

Bedeutet Hal in Formel VII Chlor oder Brom, so kann man zweckmässig Alkalijodid (wie NaJ oder KJ) zur Beschleunigung der Reaktion zusetzen. Erhöhte Temperaturen von 0 bis 220°C, bevorzugt 80-170°C, sind vorteilhaft. Zweckmässig wird das Reaktionsgemisch unter Rückfluss erhitzt.

In den Fällen, in denen in Formel VII Me für Wasserstoff steht, wird das Verfahren in Gegenwart einer Base durchgeführt. Beispiele solcher Basen sind anorganische Basen wie die Oxide, Hydroxide, Hydride, Carbonate und Hydrogencarbonate von Alkali und Erdalkalimetallen sowie z.B. tert. Amine wie Triethylamin, Triethylendiamin, Piperidin, Pyridin, 4-Dimethylaminopyridin, 4-Pyrrolidylpyridin usw.

Bei dieser und den folgenden Herstellungsvarianten können die Zwischen- und End-Produkte aus dem Reaktonsmedium isoliert und falls gewünscht, auf eine der allgemein üblichen Methoden gereinigt werden, z.B. durch Extraktion, Kristallisation, Chromatographie, Destillation usw.

Die offenen Ausgangsketale der Formel IV können z.B. durch Ketalisierungsreaktion eines 1H-1,2,4-Triazolylmethyl-phenons der Formel II in Gegenwart einer Säure mit einem Alkohol der Formel VIII,

$R_1OH$ (VIII),

worin $R_1$ die unter Formel I angegebenen Bedeutungen hat, hergestellt werden. Analoge Ketalisierungsreaktionen sind aus der US-PS 4,079,062 bekannt; man kann analog zu den dort beschriebenen Methoden vorgehen.

Bei der bevorzugten Ausführung der Ketalisierung werden beide Reaktionspartner mehrere Stunden zusammen mit einem Azeotrop-Bildner in einem der üblichen organischen Lösungsmittel unter Rückfluss erhitzt. Als Azeotrop-Bildner kommen z.B. Benzol, Toluol, Xylol, Chloroform oder Tetrachlorkohlenstoff in Frage, wobei zur Beschleunigung der Reaktion ein Zusatz einer starken Säure, wie z.B. p-Toluolsulfonsäure vorteilhaft sein kann. Verwendbare organische Lösungsmittel sind in diesem Fall z.B. aromatische Kohlenwasserstoffe, wie Benzol, Toluol, Xylol usw. und gesättigte Kohlenwasserstoffe, wie n-Hexan.

Bei der Synthese der Verbindungen der Formel I, können diese in zwei unterschiedlichen geometrischen Formen, nämlich als E- oder Z-Isomere

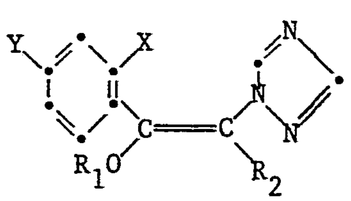

E-Form

oder

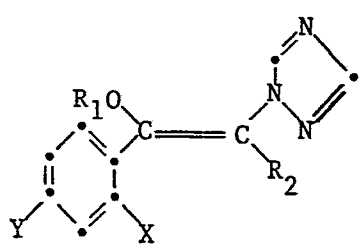

Z-Form

anfallen. Im allgemeinen entstehen Gemische beider Formen. Die Isolierung der einzelnen geometrischen Isomeren erfolgt nach üblichen Methoden, wie z.B. aufgrund unterschiedlicher Löslichkeit, durch Salzbildung, durch Craig-Verteilung oder durch chromatographische Trennverfahren bzw. durch Kombination dieser Methoden. Eine eindeutige Strukturzuordnung erfolgt aufgrund der $^{1}H$-NMR-Daten, insbesondere unter Verwendung von Verschiebungsreagenzien. Die durch diese Reagenzien bewirkten Signalverschiebungen sind um so grösser, je kleiner der räumliche Abstand zwischen dem komplexierten Zentralatom des Verschiebungsreagenzes und dem betrachteten Proton ist.

Sofern nicht speziell genannt, liegt bei der Nennung einer Verbindung der Formel I stets ein Gemisch beider geometrischen Isomeren vor. Beide Isomeren zeigen unterschiedliche mikrobizide Aktivität.

Es wurde überraschend festgestellt, dass Verbindungen der Formel I ein für praktische Bedürfnisse sehr günstiges Mikrobizid-Spektrum gegen phytopathogene Pilze und Bakterien aufweisen. Sie besitzen sehr vorteilhafte kurative, präventive und systemische Eigenschaften und lassen sich zum Schutz von Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe sind gegen die den folgenden Klassen angehörenden phytopathogenen Pilze wirksam: Ascomyceten (z.B. Venturia, Podosphaera, Erysiphe, Monilinia, Uncinula); Basidiomyceten (z.B. die Gattungen Hemileia, Rhizoctonia, Puccinia); Fungi imperfecti (z.B. Botrytis, Helminthosporium, Fusarium, Septoria, Cercospora und Alternaria) und gegen Phycomyceten wie Pythium. Ueberdies wirken die Verbindunge der Formel I systemisch. Sie können ferner als Beizmittel zur Behandlung

von Saatgut (Früchte, Knollen, Körner) und Pflanzenstecklingen zum Schutz vor Pilzinfektionen sowie gegen im Erdboden auftretende phytopathogene Pilze eingesetzt werden. Die erfindungsgemässen Wirkstoffe zeichnen sich durch besonders gute Pflanzenverträglichkeit aus.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen.

Darüberhinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für die hierun offenbarten Indikationsgebiete gelten im Rahmen dieser Erfindung beispielsweise folgende Pflanzenarten: Getreide: (Weizen, Gerste, Roggen, Hafer, Reis, Sorghum und Verwandte); Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zucerrohr, Tee, Weinreben, Hopfen, Bananen- und Naturkautschukgewächse sowie Zierpflanzen (Compositen).

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt z.B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmittel eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktibsubstanz (AS) je ha; bevorzugt 100 g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).
Ueberaus wertvolle Formulierungshilfsstoffe sind ferner Phosphorlipide.
Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder

hochdisperse saugfähige Polymerisate zugesetzt werden. Als gekörnte adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natru wie insbesondere Dolomit oder zerkleinerte Pflanzenrückstände verwendet werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z.B. das Na- oder Ca-Salz der Ligninsulfonsäure, ded Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten, vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ringwood New Jersey, 1980
Sisely and Wood, "Encyclopedia of Surface Active Agents", Chemical Publishing Co., Inc. New York, 1980.

Die agrochemischen Zubereitungen enthalten in der Regel 0,1 bis 99%, insbesondere 0,1 bis 95% Wirkstoff der Formel I, 99,9 bis 1%, insbesondere 99,8 bis 5% eines festen oder flüssigen Zusatzstoffes und 0 bis 25%, insbesondere 0,1 bis 25% eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht. Darüberhinaus werden folgende Symbole verwendet: h = Stunde; d = Tag; min =

Minute; RT = Raumtemperatur; N = Normalität; abs = absolut, wasserfrei; DMSO = Dimethylsulfoxid; DMF = Dimethylformamid.

Herstellungsbeispiele:

Beispiel 1: Herstellung von

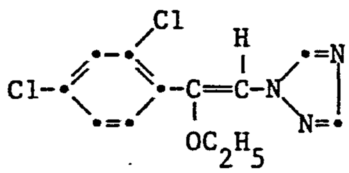

(1.2)

1-(2,4-Dichlorphenyl)-1-ethoxy-2-(1H-1,2,4-triazol-1-yl)-ethen

a) Herstellung des Ausgangsproduktes

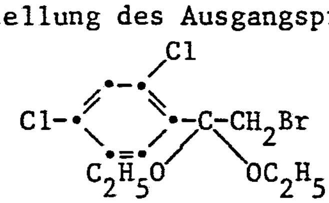

2,4-Dichlorphenacylbromid-diethylacetal

112 g 2,4-Dichlorphenacylbromid, 120 g Orthoameisensäuretriethylester und 8 g p-Toluolsulfonsäure werden in 1 Liter absolutem Ethanol 40 h unter Rückfluss erhitzt. Das Lösungsmittel wird im Vakuum entfernt, der hellgelbe ölige Rückstand mit Natriumhydrogencarbonat und Wasser gewaschen und im Vakuum von flüchtigen Bestandteilen befreit. Man erhält 140 g eines gelben Oels, das ohne weitere Reinigung weiterverarbeitet wird.

b) Herstellung eines weiteren Zwischenproduktes

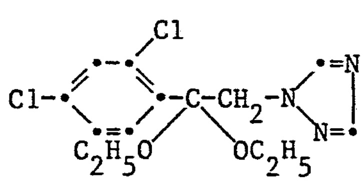

1-(2,4-Dichlorphenyl)-1,1-diethoxy-2-(1H-1,2,4-triazol-1-yl)-ethan

Zu einer Suspension von 88 g 1H-1,2,4-Triazol-Kalium in 500 ml absolutem Dimethylformamid lässt man unter Rühren eine Lösung von 140 g 2,4-Dichlorphenacylbromid-diethylacetal in 100 ml Dimethylformamid zutropfen. Die Reaktionsmischung wird nun 12 h bei einer Temperatur von 120-130° gerührt. Etwa zwei Drittel des Dimethylformamids werden im Vakuum abdestilliert und die zurückbleibende Suspension mit Eiswasser versetzt. Das sich abscheidende Oel wird mit Methylenchlorid extrahiert und nach Entfernen des Lösungsmittels im Hochvakuum destilliert. Man erhält 51 g eines fast farblosen Oels. Sdt. 155-160°/0.04 mbar.

c) Herstellung des Endproduktes

25 g des nach b) hergestellten 1-(2,4-Dichlorphenyl)-1,1-diethoxy-2-(1H-1,2,4-triazol-1-yl)-ethans werden mit 30 ml Dowtherm (= eutektische Mischung von Biphenyl und Dibenzofuran, Smp. 12-14°) und 1 g p-Toluolsulfonsäure gemischt und 5 h bei einer Badtemperatur von 220°-230° gehalten, wobei Ethanol in einem absteigenden Kühler kondensiert. Das Dowtherm wird im Vakuum abdestilliert und das zurückbleibende braune Oel in Toluol aufgenommen, mit Natriumhydrogencarbonatlösung und mit Wasser gewaschen. Ueberschüssiges Toluol wird abgedampft und das resultierende Oel durch Zugabe von Petrolether/Diethylether zur Kristallisation gebracht. Man erhält 7 g 1-(2,4-Dichlorphenyl)-1-ethoxy-(1H-1,2,4-triazol-1-yl)-ethen. Smp. 92-94°.

Beispiel 2: Herstellung von

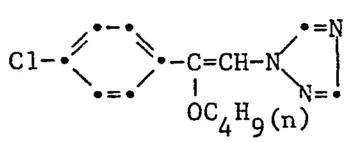

(1.7)

1-(4-Chlorphenyl)-1-n-butoxy-2-(1H-1,2,4-triazol-1-yl)-ethen

Zu einer Lösung von 17,7 g α-(1H-1,2,4-Triazol-1-yl)-4-chloracetophenon in 100 ml Hexamethylphosphorsäuretriamid gibt man unter Feuchtigkeitsausschluss 4,2 g 50%iges Natriumhydrid in Mineralöl. Nach Beendigung der Wasserstoffentwicklung lässt man 24 ml Tri-(n)-butylphosphat zu der Mischung zutropfen, steigert unter weiterem Rühren die Innentemperatur auf 75-80° und rührt weitere 10 h. Danach lässt man

das Reaktionsgemisch auf Raumtemperatur abkühlen und giesst es auf Eiswasser. Nach Extraktion mit Essigsäureethylester und Entfernung des Lösungsmittels im Vakuum bleibt ein öliges Rohprodukt zurück, das säulenchromatographisch (Silicagel/Methylenchlorid + 5% Methanol) gereinigt wird. Nach Umkristallisation aus n-Hexan erhält man 6 g farbloser Kristalle der obigen Verbindung Nr. 1.7. Smp. 75-76°.

Beispiel 3: Herstellung von

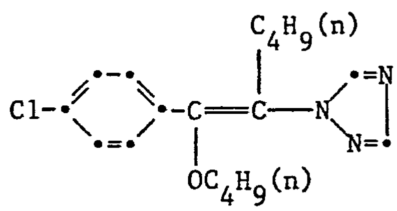

(1.26)

1-(2,4-Dichlorphenyl)-1-(n)-butoxy-2(1H-1,2,4-triazol-1-yl)-hexen (1)

Zu einer Lösung von 15 g α-(1H-1,2,4-Triazol)-1-yl)-2,4-dichloracetophenon in 50 ml DMF gibt man portionsweise 6,1 g einer 50%igen Dispersion von Natriumhydrid in Mineralöl zu. Nach dem Abklingen der Wasserstoffentwicklung lässt man 14 ml n-Butylbromid zutropfen und rührt das Reaktionsgemisch 8 h bei 50-60°. Die dunkelbraune Suspension wird auf Wasser gegossen und 3 mal mit n-Hexan und Cyclohexan extrahiert. Die vereinigten Extrakte werden mit Wasser gewaschen, über Natriumsulfat getrocknet, filtriert und im Vakuum eingeengt. Man erhält 18 g eines Oels, das aus 2 Reaktionsprodukten besteht, Ihre präparative Trennung erfolgt säulenchromatographisch (Silicagel/Methylenchlorid). Die erste Fraktion ergibt 6 g eines farblosen Oels, dessen analytische und NMRspektroskopische Daten die Struktur eines E/Z-Gemisches von 1-(2,4-Dichlorphenyl)-1-(n)-butoxy-2-(1H-1,2,4-triazol-1-yl)-hexen-1 bestätigen. Eine zweite Fraktion ergibt 6 g kristallines 1-(2,4-Dichlorphenyl)-1-(n)-butoxy-2(1H-1,2,4-triazol-1-yl)-ethen (Verbindung Nr. 1.4) vom Smp. 68-71°.

Beispiel 3a: Herstellung von

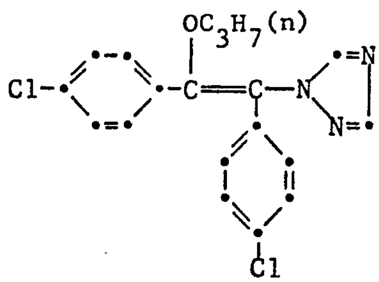

(1.32)

1-(4-Chlorphenyl)-1-n-propoxy-2(1H-1,2,4-triazol-1-yl)-2-(4-chlorphenyl)-ethen

Zu einer Lösung von 16 g α-(4-Chlorphenyl)-α-[1H-1,2,4-triazol-1-yl]-4-chloracetophenon in 150 ml absolutem DMF gibt man 4,7 g Kalium-tert.-butoxid und erwärmt das Gemisch auf 70°. Dann werden 3,3 g n-Propylbromid hinzugefügt und die Reaktionsmischung bei 70-90° 15 h gerührt. Die braune, auf RT abgekühlte Suspension wird mit Wasser verdünnt und 3 mal mit Methylenchlorid/n-Hexan (2:8) extrahiert. Die vereinigten Extrakte werden über Natriumsulfat getrocknet, filtriert und eingedampft. Nach säulenchromatographischer Reinigung über Silicagel erhält man 7 g der Verbindung Nr. 1.32 in Form von gelblichen Kristallen. Fp. 140-143°.

Auf analoge Weise werden auch die übrigen in Tabelle 1 aufgezählten Verbindungen hergestellt.

Tabelle 1: Verbindungen der Formel I

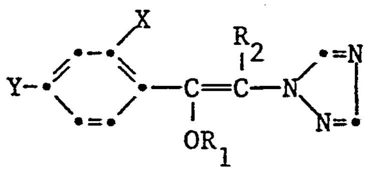

(I)

| Verb. Nr. | $R_1$ | $R_2$ | X | Y | Physikalische Daten |
|---|---|---|---|---|---|
| 1.1 | $CH_3$ | H | Cl | Cl | Fp. 98-100° |
| 1.2 | $C_2H_5$ | H | Cl | Cl | Fp. 92-94° |
| 1.3 | $C_3H_7(n)$ | H | Cl | Cl | Fp. 104-105° |
| 1.4 | $C_4H_9(n)$ | H | Cl | Cl | Fp. 68-71° |
| 1.5 | $CH(CH_3)_2$ | H | Cl | Cl | Fp. 95-96° |
| 1.6 | $C_4H_9(n)$ | H | H | Cl | Fp. 75-76° |
| 1.7 | $C_3H_7(n)$ | H | H | Cl | Fp. 82-84° |
| 1.8 | $CH(CH_3)C_2H_5$ | H | Cl | Cl | viskoses Oel |
| 1.9 | $CH(CH_3)C_3H_7(n)$ | H | Cl | Cl | viskoses Oel |
| 1.10 | $CH_2CH(CH_3)_2$ | H | Cl | Cl | Fp. 75-80° |
| 1.11 | $CH_2CH_2CH(CH_3)_2$ | H | Cl | Cl | Fp. 60-62° |
| 1.12 | $C_6H_{13}$ | H | Cl | Cl | Viskoses Oel |
| 1.13 | $CH_2CH_2OC_2H_5$ | H | Cl | Cl | Fp. 56-60° |
| 1.14 | 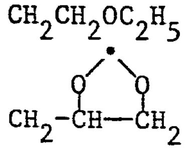 | H | Cl | Cl | |

Tabelle 1 (Fortsetzung)

| Verb. Nr. | $R_1$ | $R_2$ | X | Y | Physikalische Daten |
|---|---|---|---|---|---|
| 1.15 | 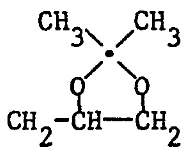 | H | Cl | Cl | |
| 1.16 | 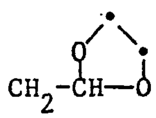 | H | Cl | Cl | Fp. 128-129° |
| 1.17 | 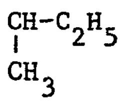 | H | H | F | Fp. 53-60° |
| 1.18 | $CH_2CH_2CH(CH_3)_2$ | H | H | F | |
| 1.19 | $C_8H_{17}$ | H | H | F | |
| 1.20 | $CH_3$ | H | H | $C_6H_5$ | Fp. 104-106° |
| 1.21 | $C_4H_9$(n) | H | H | $C_6H_5$ | |
| 1.22 | $C_4H_9$ | $C_2H_5$ | Cl | Cl | viskoses Oel |
| 1.23 | $CH_3$ | $C_4H_9$(n) | Cl | Cl | |
| 1.24 | $CH_3$ | $C_2H_5$ | Cl | Cl | viskoses Oel |
| 1.25 | $C_2H_5$ | $C_2H_5$ | Cl | Cl | |
| 1.26 | $C_4H_9$(n) | $C_4H_9$(n) | Cl | Cl | viskoses Oel |
| 1.27 | $CH_2CH=CH_2$ | $CH_2CH=CH_2$ | Cl | Cl | viskoses Oel |
| 1.28 | $CH_2C\equiv CH$ | $CH_2C\equiv CH$ | Cl | Cl | viskoses Oel |
| 1.29 | $CH_2C\equiv CH$ | $CH_2C\equiv CH$ | H | Cl | viskoses Oel |
| 1.30 | $CH_3$ | $CH_2CH=CH_2$ | Cl | Cl | |
| 1.31 | $C_4H_9$(n) | $C_6H_4Cl(4)$ | H | Cl | Fp. 85-87° |
| 1.32 | $C_3H_7$(n) | $C_6H_4Cl(4)$ | H | Cl | Fp. 140-143° |

Formulierungsbeispiele für flüssige Wirkstoffe der Formel I

(% = Gewichtsprozent)

| 4. Emulsions-Konzentrate | a) | b) | c) |
|---|---|---|---|
| Wirkstoff aus Tabelle 1 | 25% | 40% | 50% |
| Ca-Dodecylbenzolsulfonat | 5% | 8% | 6% |
| Ricinusöl-polyethylenglykolether (36 Mol Ethylenoxid) | 5% | - | - |
| Tributylphenoyl-polyethylenglykolether (30 Mol Ethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 5. Lösungen | a) | b) | c) | d) |
|---|---|---|---|---|
| Wirkstoff aus Tabelle 1 | 80% | 10% | 5% | 95% |
| Ethylenglykol-monomethyl-ether | 20% | - | - | - |
| Polyethylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

(MG = Molekulargewicht)

Die Lösungen sind zur Anwendung in Form kleinster Tropfen geeignet.

| 6. Granulate | a) | b) |
|---|---|---|
| Wirkstoff aus Tabelle 1 | 5% | 10% |
| Kaolin | 94% | - |
| Hochdisperse Kieselsäure | 1% | - |
| Attapulgit | - | 94% |

Der Wirkstoff wird in Methylenchlorid gelöst, auf den Träger aufgesprüht und das Lösungsmittel anschliessend im Vakuum abgedampft.

7. Stäubemittel

| | a) | b) |
|---|---|---|
| Wirkstoff aus Tabelle 1 | 2% | 5% |
| Hochdisperse Kieselsäure | 1% | 5% |
| Talkum | 97% | - |
| Kaolin | - | 90% |

Durch inniges Vermischen auf Trägerstoffe mit dem Wirkstoff erhält man gebrauchsfertige Stäubemittel.

Formulierungsbeispiele für feste Wirkstoffe der Formel I

(% = Gewichtsprozent)

8. Spritzpulver

| | a) | b) | c) |
|---|---|---|---|
| Wirkstoff aus Tabelle 1 | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyethylenglykolether (7-8 Mol Ethylenoxid) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

9. Emulsions-Konzentrat

| | |
|---|---|
| Wirkstoff aus Tabelle 1 | 10% |
| Octylphenolpolyethylenglykolether (4-5 Mol Ethylenoxid) | 3% |
| Ca-Dodecylbenzolsulfonat | 3% |
| Ricinusölpolyglykolether (35 Mol Ethylenoxid) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 10. Stäubemittel | a) | b) |
|---|---|---|
| Wirkstoff aus Tabelle 1 | 5% | 8% |
| Talkum | 95% | - |
| Kaolin | - | 92% |

Man erhält anwendungsfertige Stäubemittel, indem der Wirkstoff mit den Trägern vermischt und auf einer geeigneten Mühle vermahlen wird.

| 11. Extruder Granulat | |
|---|---|
| Wirkstoff aus Tabelle 1 | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

| 12. Umhüllungs Granulat | |
|---|---|
| Wirkstoff aus Tabelle 1 | 3% |
| Polyethylenglykol (M G 200) | 3% |
| Kaolin | 94% |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

13. <u>Suspensions-Konzentrat</u>

| | |
|---|---|
| Wirkstoff aus Tabelle 1 | 40% |
| Ethylenglykol | 10% |
| Nonylphenolpolyethylenglykolether (15 Mol Ethylenoxid) | 6% |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Bei den nachfolgenden biologischen Untersuchungen werden unter anderen die nächstvergleichbaren, aus der Literatur bekannten Substanzen A und B vergleichend mitgeprüft:

Aus der DE-OS 29 31 755:

A 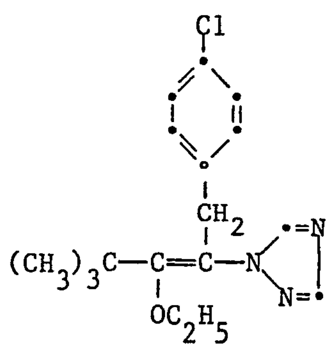 Seite 27; Beispiel 1; Z/E-Gemisch

Aus der DE-OS 26 10 022:

B 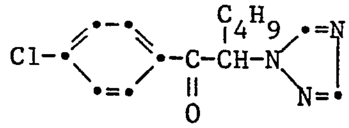 Seite 5; Verbindung Nr. 1

Biologische Beispiele:

Beispiel 14: Wirkung gegen Puccinia graminis auf Weizen

a) Residual-protektive Wirkung

Weizenpflanzen wurden 6 Tage nach der Aussaat mit einer aus Spritzpulver des Wirkstoffes hergestellten Spritzbrühe (0,002% Aktivsubstanz) besprüht. Nach 24 Stunden wurden die behandelten Pflanzen mit einer Ureidosporensuspension des Pilzes infiziert. Nach einer Inkubation während 48 Stunden bei 95-100% relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion.

b) Systemische Wirkung

Zu Weizenpflanzen wurde 5 Tage nach der Aussaat eine aus Spritzpulver des Wirkstoffes hergestellte Spritzbrühe gegossen (0,002% Aktivsubstanz bezogen auf das Bodenvolumen). Nach 48 Stunden wurden die behandelten Pflanzen mit einer Uredosporensuspension des Pilzes infiziert. Nach einer Inkubation während 48 Stunden bei 95-100% relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion.

Verbindungen aus Tabelle 1 zeigten gegen Puccinia-Pilze eine gute Wirkung. Unbehandelte aber infizierte Kontrollpflanzen zeigten einen Puccinia-Befall von 100%. Bei einer Prüfkonzentration von 0,002% hemmten die Verbindungen Nr. 1.3, 1.6, 1.9, 1.22 und 1.26 den Puccinia-Befall vollständig (0-5%). Bei 0,006% Aktivsubstanz bewirkten auch die übrigen Substanzen der Tabelle 1 eine 0-5%ige Hemmung des Befalls. Bei der niedrigen Prüfkonzentration von 0,002% bewirkte die Vergleichsverbindung A eine Hemmung von 30-40% und B eine Hemmung von 50%.

Beispiel 15: Wirkung gegen Cercospora arachidicola auf Erdnusspflanzen

a) Residual-protektive Wirkung

10-15 cm hohe Erdnusspflanzen wurden mit einer aus Spritzpulver der Wirksubstanz hergestellten Spritzbrühe (0,002 Aktivsubstanz besprüht und 48 Stunden später mit einer Konodiensuspension des Pilzes infiziert. Die infizierten Pflanzen wurden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

b) Systemische Wirkung

Zu 10-15 cm hohen Erdnusspflanzen wurde eine aus Spritzpulver des Wirkstoffes hergestellte Spritzbrühe gegossen (0,006% Aktivsubstanz bezogen auf das Erdvolumen). Nach 48 Stunden wurden die behandelten Pflanzen mit einer Konidiensuspension des Pilzes infiziert und 72 Stunden bei ca. 21°C und hoher Luftfeuchtigkeit inkubiert. Anschliessend wurden die Pflanzen im Gewächshaus aufgestellt und nach 11 Tagen der Pilzbefall beurteilt.

Im Vergleich zu unbehandelten, aber infizierten Kontrollpflanzen (Anzahl und Grösse der Flecken = 100%), zeigten Erdnusspflanzen, die mit Wirkstoffen aus Tabelle 1 behandelt wurden, einen stark reduzierten Cercospora-Befall. So verhinderten die Verbindungen Nr. 1.1 und 1.4 in obigen Versuchen das Auftreten von Flecken fast vollständig (0-5%).

Dagegen bewirkten die nächstvergleichbaren Verbindungen A und B des Standes der Technik bei obigen Prüfkonzentrationen keine Hemmung des Cercosporabefalls. Selbst bei einer Erhöhung der Prüfkonzentration auf 0,02% war keinerlei Befalls-Reduktion zu beobachten. Bei Behandlung mit Vergleichssubstanz B wurden phytotoxische Effekte beobachtet.

Beispiel 16: Wirkung gegen Erysiphae graminis auf Gerste

a) Residual-protektive Wirkung

Ca. 8 cm hohe Gerstenpflanzen wurden mit einer aus Spritzpulver des Wirkstoffes hergestellten Spritzbrühe (0,0002% Aktivsubstanz) besprüht. Nach 3-4 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellte Spritzbrühe gegossen (0,006% Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

Verbindungen der Formel I zeigten gute Wirkung gegen Erysiph-Pilze. Unbehandelte, aber infizierte Kontrollpflanzen zeigten einen Erysiphe-Befall von 100%. Unter anderen Verbindungen aus Tabelle i hemmten die Verbindungen Nr. 1.1 bis 1.4, 1.6, 1.8, 1.9, 1.22, 1.31 und 1.32 den Pilzbefall bei Gerste auf 0 bis 5%, insbesondere die Verbindungen Nr. 1.3 und 1.4 bewirkten selbst bei noch tieferen Prüfkonzentrationen von 0,00002% eine vollständige Hemmung des Erysiphe-Befalls. Eine vergleichbar gute Wirkung konnte mit den Substanzen A und B des Standes der Technik erst bei erhöhten Prüfkonzentrationen von 0,02% beobachtet werden. Substanz B bewirkte zusätzlich eine deutliche Schädigung der Testpflanzen.

Beispiel 17: Residual-protektive Wirkung gegen Venturia inaequalis auf Apfeltrieben.

Apfelstecklinge mit 10 - 20 cm langen Frischtrieben wurden mit einer aus Spritzpulver des Wirkstoffes hergestellten Spritzbrühe (0,006% Aktibsubstanz) besprüht. Nach 24 Stunden wurden die behandelten Pflanzen mit einer Konidiensuspension des Pilzes infiziert. Die Pflanzen wurden dann während 5 Tagen bei 90-100% relativer Luftfeuchtigkeit inkubiert und während 10 weiteren Tagen in einem Gewächshaus bei 20-24°C aufgestellt. Der Schorfbefall wurde 15 Tage nach der Infektion beurteilt. Verbindung Nr. 1.4 und andere hemmten den Krankheitsfefall auf weniger als 10%. Unbehandelte aber infizierte Triebe zeigten einen 100%igen Venuria-Befall vollständig. Vergleichssubstanz B zeigte keine Venturia-Wirkung; Substanz A erst bei einer Prüfkonzentration von 0,02%.

Beispiel 18: Wirkung gegen Bortytis cinerea auf Bohnen
Residual protektive Wirkung

Ca. 10 cm hohe Bohnen-Pflanzen wurden mit einer aus Spritzpulver des Wirkstoffes hergestellten Spritzbrühe (0,002% Aktivsubstanz) besprüht. Nach 48 Stunden wurden die behandelten Pflanzen mit einer Konidiensuspension des Pilzes infiziert. Nach einer Inkubation der infizierten Pflanzen während 3 Tagen bei 95-100% relativer Luftfeuchtigkeit und 21°C erfolgte die Beurteilung des Pilzbefalls. Die Verbindungen aus Tabelle 1 hemmten in vielen Fällen die Pilzinfektion sehr stark. Bei einer Konzentration von 0,002% erwiesen sich z.B. die Verbindungen Nr. 1.9 und 1.22 als voll wirksam (Krankheitsbefall 0 bis 5%). Bei 0,006% zeigten auch die Verbindungen Nr. 1.8 und 1.28 volle Wirkung (0 bis 5% Befall). Der Botrytis-Befall unbehandelter aber infizierter Kontrollpflanzen betrug dagegen 100%. Die nächstvergleichbaren Verbindungen A und B des Standes der Technik zeigten gegen Botrytis-Pilze keinerlei Befallsreduktion. Substanz B bewirkte lediglich eine sichtbare Schädigung der Testpflanzen.

Die biologischen Untersuchungen (Beispiele Nr. 14 bis 18) zeigen mit grosser Deutlichkeit die Ueberlegenheit der Verbindungen vorliegender Erfindung in der Bekämpfung von Pilzkrankheiten an Kulturpflanzen im Vergleich zu vorbekannten Substanzen ähnlicher Konstitution.

Patentansprüche

1. Verbindungen der allgemeinen Formel I

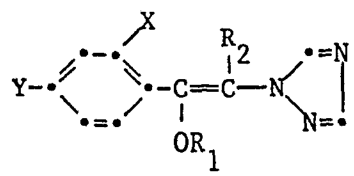

(I),

worin

X für Wasserstoff oder Halogen steht;

Y Wasserstoff, Halogen oder ein gegebenenfalls durch ein oder zwei Halogenatome substituiertes Phenyl bedeutet;

$R_1$ für $C_1$-$C_8$-Alkyl, $C_3$-$C_5$-Alkenyl, $C_3$-Alkinyl, einen Niederalkoxyalkylrest mit maximal 8 Kohlenstoffatomen oder für eine der Gruppen

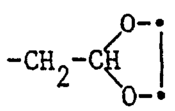 oder 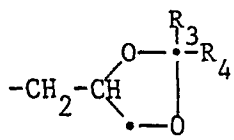 steht,

wobei $R_3$ und $R_4$ unabhängig voneinander Wasserstoff oder Methyl bedeuten; und $R_2$ für Wasserstoff, $C_1$-$C_4$-Alkyl, $C_3$-Alkenyl, $C_3$-Alkinyl oder ein gegebenenfalls ein- oder zweifach durch Halogen, $C_1$-$C_3$Alkyl oder $C_1$-$C_3$-Alkoxy substituiertes Phenyl steht; unter Einschluss ihres Säureadditionssalze, quaternären Azoliumsalze und Metallkomplexe.

2. Verbindungen der Formel I, worin X für Wasserstoff, Fluor, Chlor, Brom oder Jod steht; Y Wasserstoff, Fluor, Chlor, Brom, Jod, $C_6H_5$, $C_6H_4Hal(2)$, $C_6H_4Hal(4)$, $C_6H_3Hal_2(2,4)$ oder $C_6H_3Hal_2(3,4)$ bedeutet, wobei Hal für Fluor, Chlor, Brom oder Jod steht; $R_1$ für $C_1$-$C_5$-Alkyl, $CH_2CH{=}CH_2$, $CH_2C{\equiv}CH$, $CH_2OCH_3$, $CH_2OC_2H_5$, $CH_2OC_3H_7$, $CH_2CH_2OCH_3$, $CH_2CH_2OC_2H_5$, 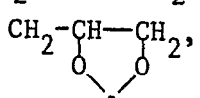, 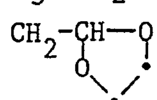 oder 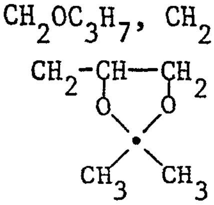 steht und $R_2$ Wasserstoff, $C_1$-$C_4$-Alkyl, $CH_2CH{=}CH_2$, $CH_2C{\equiv}CH$, $C_6H_3Cl_2(2,4)$ oder $C_6H_4Cl(4)$ bedeutet.

3. Verbindungen der Formel I, worin X für Wasserstoff, Fluor, Chlor oder Brom steht, Y Wasserstoff, Fluor, Chlor, $C_6H_5$, $C_6H_4Cl(2)$, $C_6H_4Cl(4)$, $C_6H_3Cl_2(2,4)$ oder $C_6H_3Cl_2(3,4)$ bedeutet; $R_1$ für $C_1$-$C_4$-Alkyl, $CH_2CH{=}CH_2$, $CH_2C{\equiv}CH$, $CH_2OCH_3$, $CH_2OC_2H_5$ oder $CH_2CH_2OCH_3$ steht und $R_2$ Wasserstoff oder $C_1$-$C_4$-Alkyl bedeutet.

4. Verbindungen der Formel I, worin X für Wasserstoff oder Chlor; Y für Wasserstoff, Fluor, Chlor oder $C_6H_5$ stehen; $R_1$ Methyl, Ethyl, n-Proypl, iso-Propyl, n-Butyl, sek.-Butyl, tert.-Butyl, $CH_2OCH_3$, $CH_2OC_2H_5$ oder $CH_2CH_2OCH_3$ bedeutet und $R_2$ für Wasserstoff, Methyl, Ethyl, n-Propyl, iso-Propyl, n-Butyl oder sek.-Butyl steht.

5. Verbindungen der Formel I, worin X für Chlor steht: Y Fluor oder Chlor bedeutet; $R_1$ für Methyl, Ethyl, n-Propyl oder n-Butyl steht und $R_2$ Wasserstoff bedeutet.

6. Eine Verbindung ausgewählt aus der Reihe:

1-(2,4-Dichlorphenyl)-1-n-propyloxy-2-(1H-1,2,4-triazol-1-yl)-ethen,
1-(2,4-Dichlorphenyl)-1-n-butyloxy-2-(1H-1,2,4-triazol-1-yl)-ethen,
1-(2,4-Dichlorphenyl)-1-(1'-methylbutoxy)-2-(1H-1,2,4-triazol-1-yl)-ethen,
1-(2,4-Dichlorphenyl)-1-n-butyloxy-2-(1H-1,2,4-triazol-1-yl)-buten,
1-(2,4-Dichlorphenyl)-1-n-butoxy-2-(1H-1,2,4-triazol-1-yl)-hexen.

7. Verfahren zur Herstellung von Verbindungen der in Anspruch 1 definierten Formel I, dadurch gekennzeichnet, dass man

A) ein α-1H-1,2,4-Triazolylphenon der Formel II,

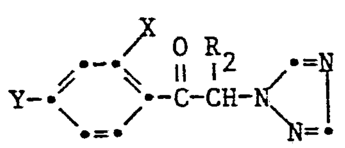

(II),

worin X, Y und $R_2$ die unter Formel I angegebenen Bedeutungen haben, mit einer Verbindung der Formel III,

$$R_1\text{-}Z \qquad \text{(III)},$$

worin $R_1$ wie unter Formel I definiert ist und Z für eine der folgenden Abgangsgruppierungen -Chlor, -Brom, $R_1SO_4^{\ominus}$ oder $(R_1O)_2(O)P{=}O^{\ominus}$ steht, worin $R_1$ ebenfalls wie unter Formel I definiert ist, in Anwesenheit einer Base und vorteilhafterweise in Anwesenheit eines polaren, aprotischen Lösungsmittels oder eines wässrig-organischen Zweiphasenmediums in Gegenwart eines üblichen Phasentransferkatalysators reagieren lässt; oder indem man in den Fällen in denen $R_2$ für Wasserstoff steht,

B) ein offenes Acetal der Formel IV,

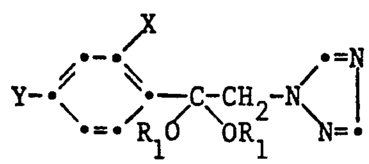 (IV),

worin X, Y und $R_1$ die unter Formel I angegebenen Bedeutungen haben, in einem hochsiedenden Lösungsmittel bei +100° bis 220°C einer Thermolyse unterwirft.

8. Schädlingsbekämpfungsmittel zur Bekämpfung oder Verhütung eines Befalls durch Mikroorganismen, dadurch gekennzeichnet, dass es als mindestens eine aktive Komponente eine Verbindung der Formel I gemäss Anspruch 1 enthält.

9. Mittel gemäss Anspruch 8, dadurch gekennzeichnet, dass es als mindestens eine aktive Komponente eine Verbindung der Formel I gemäss Anspruch 2 enthält.

10. Mittel zur Bekämpfung von Mikroorganismen gemäss Anspruch 8, dadurch gekennzeichnet, dass es als mindestens eine aktive Komponente eine Verbindung der Formel I gemäss Anspruch 3 enthält.

11. Mittel zur Bekämpfung von Mikroorganismen gemäss Anspruch 8, dadurch gekennzeichnet, dass es als mindestens eine aktive Komponente eine Verbindung der Formel I gemäss einem der Ansprüche 4 bis 6 enthält.

12. Mittel nach einem der Ansprüche 8 bis 11, dadurch gekennzeichnet, dass es 0,1 bis 99% eines Wirkstoffes der Formel I, 99,9 bis 1% eines festen oder flüssigen Zusatzstoffes und 0 bis 25% eines Tensides enthält.

13. Mittel nach Anspruch 12, dadurch gekennzeichnet, dass es 0,1 bis 95% eines Wirkstoffes der Formel I, 99,8 bis 5% eines festen oder flüssigen Zusatzstoffes und 0,1 bis 25% eines Tensides enthält.

14. Verfahren zur Herstellung eines agrochemischen Mittels, dadurch gekennzeichnet, dass mindestens eine Verbindung der Formel I gemäss einem der Ansprüche 1 bis 6 mit geeigneten festen oder flüssigen Zusatzstoffen und Tensiden innig vermischt.

15. Verfahren zur Bekämpfung oder Verhütung eines Befalls von Kulturpflanzen durch phytopathogene Mikroorganismen, dadurch gekennzeichnet, dass man eine Verbindung der Formel I gemäss einem der Ansprüche 1 bis 6 auf die Pflanze oder deren Standort appliziert.

16. Verwendung von Verbindungen der Formel I gemäss Anspruch 1 zur Bekämpfung und/oder zur präventiven Verhütung eines Befalls von Mikroorganismen.

17. Verwendung nach Anspruch 16 von Verbindungen der Formel I gemäss einem der Ansprüche 2 bis 6.

18. Verwendung gemäss Anspruch 16, dadurch gekennzeichnet, dass es sich bei den Mikroorganismen um phytopathogene Pilze handelt.

19. Verwendung gemäss Anspruch 16 gegen Pilze aus den Klassen Ascomycetes, Basidiomycetes oder Fungi imperfecti.

Europäisches Patentamt

# EUROPÄISCHER RECHERCHENBERICHT

0079856
Nummer der Anmeldung

EP 82 81 0477

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 3) |
|---|---|---|---|
| | --- | | C 07 D 249/08 |
| A | EP-A-0 028 363 (BASF)<br>* Ansprüche *<br>----- | 1-5,7-19 | C 07 D 405/00<br>A 01 N 43/64 //<br>C 07 C 43/174 |
| | | | **RECHERCHIERTE SACHGEBIETE (Int Cl 3)** |
| | | | C 07 D 249/00<br>C 07 D 405/00 |

Der vorliegende Recherchenbericht wurde für alle Patentansprüche erstellt.

| Recherchenort | Abschlußdatum der Recherche | Prüfer |
|---|---|---|
| DEN HAAG | 16-02-1983 | CREMERS K. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82